# NEW EUROPEAN PATENT SPECIFICATION

(11) **EP 0 639 070 B2**
(45) Date of publication and mention of the opposition decision: **08.09.2010**
(45) Mention of the grant of the patent: 03.08.2005
(21) Application number: 93911061.5
(22) Date of filing: 04.05.1993
(51) Int. Cl.: A61K 9/00

(54) **USE OF BORATE-POLYOL COMPLEXES IN OPHTHALMIC COMPOSITIONS**
ANWENDUNG VON BORAL-POLYOL-KOMPLEXEN FÜR OPHTHALMISCHE ARZNEIZUSAMMENSETZUNGEN
UTILISATION DE COMPLEXES BORATE-POLYOL DANS DES COMPOSITIONS OPHTALMIQUES

(30) Priority: 06.05.1992 US 879435
(43) Date of publication of application: 22.02.1995
(73) Proprietor: ALCON LABORATORIES, INC., Fort Worth TX 76134-2099 (US)
(72) Inventor: CHOWHAN, Masood, Arlington, TX 76014 (US); Dassanayake, Nissanke, Lakshman, Arlington, TX 76013 (US)
(74) Representative: Best, Michael
(86) International application number: PCT/US1993/004226
(87) International publication number: WO 1993/021903

(56) References cited:
- EP-A1- 0 109 561
- EP-A1- 0 436 726
- EP-A2- 0 508 381
- FR-A2- 2 230 358
- HOULSBY, R. DENNIS ET AL: 'Antimicrobial Activity of Borate-Buffered Solutions' ANTIMICROBIAL AGENTS AND CHEMOTHERAPY vol. 29, no. 5, May 1986, pages 803 - 806

## Description

This invention relates to the use of borate-polyol complexes in ophthalmic compositions. In particular, these complexes are useful as antimicrobial agents in aqueous ophthalmic compositions, including those ophthalmic compositions containing polyvinyl alcohol.

Ophthalmic compositions often include such adjuvants as buffers, tonicity-adjusting agents and viscosity-enhancing agents. Such adjuvants are discussed, for example, in EP-A-109561, FR-A-1473318 and EP-A-0436726.

Ophthalmic compositions are generally formulated to have a pH between about 4.0 and 8.0. To achieve a pH in this range and to maintain the pH for optimal stability during the shelf life of the composition, a buffer is often included. Borate is the buffer of choice for use in ophthalmic compositions, since it has some inherent antimicrobial activity and often enhances the activity of antimicrobials. However, when polyvinyl alcohol(PVA) is also an ingredient in the composition, borate and PVA form a water-insoluble complex which precipitates out of solution and acts as an irritant in the eye. This incompatibility of borate and PVA in contact lens solutions is well-known, and has been discussed, for example, in an article by P.L. Rakow in Contact Lens Forum, (June 1988), pages 41-46. Moreover, borate buffer cannot be effectively used below pH 7.0 due to its low buffering capacity to lower pH.

Since borate is incompatible with PVA, ophthalmic compositions containing PVA are generally buffered with acetate, phosphate or other buffers. There are disadvantages of using these alternative buffers: for example, acetate is a weak buffer (pKₐ of about 4.5), so a relatively large amount is needed; on the other hand, phosphate is a good buffer but, when used in concentrations generally found in ophthalmic formulations, it reduces the antimicrobial activity of preservatives.

It is well known that small organic compounds, such as benzalkonium chloride (BAC), chlorhexidine, thimerosal have excellent antimicrobial activity; however, it is now known that these small organic antimicrobials are often toxic to the sensitive tissues of the eye and can accumulate in contact lenses, particularly soft, hydrophilic contact lenses. More recently, polymeric antimicrobials such as Polyquad® (polyquaternium-1) and Dymed® (polyhexamethylene biguanide) have been used in contact lens care products as disinfectants and preservatives. While these polymeric antimicrobials exhibit a broad spectrum of antimicrobial activity, they generally have relatively weak antifungal activity, especially against *Aspergillus niger* and *Aspergillus fumigatus*.

In EP-A-0109561 there is disclosed an ophthalmic preparation for the treatment of glaucoma by the application to the glaucomatous eye of a celiprolol salt in a pharmaceutically acceptable ophthalmic carrier. In an embodiment of the compositions described in this specification, there is provided a buffer which can include a borate compound.

In FR-A-2230358 there is disclosed a substantially clear sterile aqueous solution containing, as active ingredient, a therapeutically useful proportion of 1,3-bis-(2-carboxychromon-5-xyloxy)-propan-2-ol and an additional compound which is therapeutically useful in the eye. The additional compound may comprise boric acid/sodium borate.

In EP-A-0436726 there is disclosed a method of photostabilising an eyewash containing a photo-unstable medicine as an ingredient, which comprises adding thereto boric acid and/or borax and a polyhydric alcohol.

A need therefore exists for ophthalmic compositions which have an optimal pH for stability and efficacy, but whose antimicrobial efficacy is not compromised.

The ophthalmic compositions utilised in the present invention comprise borate-polyol complexes which have surprisingly been found to be increased in antimicrobial activity as compared to boric acid or its salts, particularly with respect to organisms such as *A. niger.* Moreover, these complexes unexpectedly increase the antimicrobial efficacy of other antimicrobial agents when used in combination.

The borate-polyol complexes are formed by mixing boric acid and/or its salts with polyols, such as mannitol, glycerin or propylene glycol, in an aqueous solution. The resultant solution may then be used as antimicrobial agent in aqueous ophthalmic compositions, even where such compositions also contain PVA. The borate-polyol complexes are also useful in unpreserved saline solutions.

According to one aspect, the invention includes the use of a water-soluble borate-polyol complex, said polyol having at least two adjacent -OH groups which are not in trans configuration relative to each other, as an ophthalmic antimicrobial agent in an aqueous ophthalmic composition, wherein the polyol is selected from the group consisting of mannitol, glycerin, propyleneglycol and sorbitol.

In another aspect, the invention provides the use of a water-soluble borate-polyol complex, said polyol having at least two adjacent -OH groups which are not in trans configuration relative to each other, as an ophthalmic antimicrobial agent against *A. niger* in an aqueous composition.

The borate-polyol complex can be included in an aqueous ophthalmic composition for treating contact lenses. Preferably, 0.5 to 6.0 wt% of the borate-polyol complex is included in an aqueous ophthalmic composition, the complex containing borate and polyol in a molar ratio of 1:0.1 to 1:10.

The borate-polyol complex can be included in an aqueous ophthalmic composition which is a solution for disinfecting contact lenses, to enhance the antimicrobial activity of the solution. The borate-polyol complex can also be included in an aqueous ophthalmic composition which is an unpreserved saline solution for treating contact lenses, to enhance the antimicrobial activity of the saline solution.

In the compositions used according to the invention, the borate-polyol complex can be present in an amount of from 0.5 to 6.0% by weight of said composition to enhance antimicrobial activity of the composition against *A. niger.*

If desired, the composition can further comprise an ophthalmically acceptable antimicrobial agent such as those disclosed herein. The composition may be, for example, a solution for disinfecting contact lenses, or an unpreserved saline solution.

The invention also provides the use of the composition as an antimicrobial composition against *A. niger,* and its use for the treatment of contact lenses.

The borate-polyol complexes utilised in the present invention are particularly useful as adjunctive disinfecting agents in contact lens disinfecting solutions containing monomeric quaternary ammonium compounds (e.g. benzalkonium chloride) or biguanides (e.g. chlorhexidine) or polymeric antimicrobials, such as polymeric quaternary ammonium compounds (e.g. Polyquad®, Alcon Laboratories, Inc., Fort Worth, Texas) or polymeric biguanides (e.g. Dymed®, Bausch & Lomb, Rochester, New York).

The compositions utilised in the present invention may optionally contain PVA (polyvinylalcohol); such compositions are particularly useful in contact lens care products which are targeted for wearers of rigid gas-permeable contact lenses (RGPs), who often complain of discomfort. PVA is a viscosity enhancer and is used extensively in all types of RGP products in order to improve the comfort and wearing time of RGPs. PVA is also extensively used as a viscosity enhancer for pharmaceutical ophthalmic compositions such as eye drops, gels or ocular inserts.

As used herein, the term "borate" shall refer to boric acid, salts of boric acid and other pharmaceutically acceptable borates, or combinations thereof. Most suitable are: boric acid, sodium borate, potassium borate, calcium borate, magnesium borate, manganese borate, and other such borate salts.

As used herein, and unless otherwise indicated, the term "polyol" shall refer to any compound having at least two adjacent -OH groups which are not in trans configuration relative to each other. The polyols can be linear or circular, substituted or unsubstituted, or mixtures thereof, so long as the resultant complex is water-soluble and pharmaceutically acceptable. Such compounds include sugars, sugar alcohols, sugar acids and uronic acids. Preferred polyols are sugars, sugar alcohols and sugar acids, including, mannitol, glycerin, propylene glycol and sorbitol. Especially preferred polyols are mannitol and glycerin; most preferred is mannitol.

The water-soluble borate-polyol complexes utilised in the present invention may be formed by mixing borate with the polyol(s) of choice in an aqueous solution. These complexes can be used in conjunction with other known preservatives and disinfectants to meet preservative efficacy and disinfection standards. In such compositions, the molar ratio of borate to polyol is generally between about 1:0.1 and about 1:10, and is preferably between about 1:0.25 and about 1:2.5. The borate-polyol complexes may also be used in unpreserved salines to meet preservative efficacy standards. In these unpreserved salines, the molar ratio of borate to polyol is generally between about 1:0.1 and about 1:1, and is especially between about 1:0.25 and about 1:0.75. Some borate-polyol complexes, such as potassium borotartrate, are commercially available.

The borate-polyol complexes are utilised in the present invention in an amount between about 0.5 to about 6.0 percent by weight (wt%), preferably between about 1.0 to about 3.0 wt%, more preferably between about 1.0 to about 2.5 wt%, and most preferably between about 1.0 to about 2.0 wt %. The optimum amount, however, will depend upon the complexity of the product, since potential interactions may occur with the other components of a composition. Such optimum amount can be readily determined by one skilled in the formulatory arts.

The compositions utilised in the present invention useful with RGPs or compositions such as eye drops, gels or ocular inserts will preferably also contain PVA (polyvinylalcohol) or other viscosity-enhancing polymers, such as cellulosic polymers or carboxy vinyl polymers. PVA is available in a number of grades, each differing in degree of polymerisation, percent of hydrolysis, and residual acetate content. Such differences affect the physical and chemical behaviour of the different grades. PVA can be divided into two broad categories, i.e., completely hydrolyzed and partially hydrolyzed. Those containing 4% residual acetate content or less are referred to as completely hydrolyzed. Partially hydrolyzed grades usually contain 20% or more residual acetate. The molecular weight of PVA's vary from 20,000 to 200,000. In general, PVA used in ophthalmic products has an average molecular weight in the range of 30,000 to 100,000 with 11% to 15% residual acetate. Compositions of the present invention generally contain such types of PVA at a concentration less than about 10.0 wt%, preferably between about 0.1 and about 1.4 wt% and most preferably at a concentration of about 0.75 wt%.

### EXAMPLE 1

The water-soluble borate-polyol complexes used according to the present invention may be prepared as illustrated below.

| **INGREDIENT** | **FORMULATION (% weight/volume)** | | | | | | | |
|---|---|---|---|---|---|---|---|---|
| | **A** | **B** | **C** | **D** | **E** | **F** | **G** | **H** |
| Boric acid | 0.35 | 0.35 | 0.35 | 0.35 | 0.35 | 0.35 | 0.35 | 0.35 |
| Sodium borate | 0.11 | 0.11 | 0.11 | 0.11 | 0.11 | 0.11 | 0.11 | 0.11 |
| Mannitol | 0.5 | 1.0 | 1.5 | 2.0 | - | - | - | - |
| Glycerin | - | - | - | - | 0.5 | 1.0 | 1.5 | 2.0 |
| Na₂EDTA | 0.1 | 0.1 | 0.1 | 0.1 | 0.1 | 0.1 | 0.1 | 0.1 |
| Purified water | q.s. | q.s. | q.s. | q.s. | q.s. | q.s. | q.s. | q.s. |
| HCl/NaOH | pH 7.4 | pH 7.4 | pH 7.4 | pH 7.4 | pH 7.4 | pH 7.4 | pH 7.4 | pH 7.4 |
| Polyquad® | 0.001 + 10% xs | 0.001 + 10% xs | 0.001 + 10% xs | 0.001 + 10% xs | 0.001 + 10% xs | 0.001 + 10% xs | 0.001 + 10% xs | 0.001 + 10% xs |

### Preparation:

Formulations A - H were prepared as follows. Tubular, labeled and calibrated 150 milliliter (mL) beakers were each filled with about 90 mL of purified water. Boric acid, sodium borate and mannitol or glycerin were then added and dissolved by stirring the solution for about 25 minutes. At this time, disodium EDTA (ethylene diamine tetraacetic acid) was added with stirring. Purified water was added to bring the solutions almost to 100% (100 mL), pH was adjusted to approximately 7.4 and the osmolality was measured. Polyquad® was then added and the volume brought to 100% by the addition of purified water. pH was again measured and adjusted, if necessary, and the osmolality was measured again.

It is not always necessary to have an isotonic solution; however, if there is a need to have an isotonic solution, the osmolality can be adjusted by incorporating polyol with OH groups in *trans* position, sodium chloride, potassium chloride, calcium chloride or other osmolality building agents which are generally acceptable in ophthalmic formulations and known to those skilled in the art.

### EXAMPLE 2

Aqueous ophthalmic compositions used according to the present invention may be prepared using the formulations illustrated below.

| **INGREDIENT** | **FORMULATION (percent by weight)** | | | | | | | | |
|---|---|---|---|---|---|---|---|---|---|
| | **1** | **2** | **3** | **4** | **5** | **6** | **7** | **8** | **9** |
| PVA | 0.75 | 1.4 | 0.75 | 0.75 | 0.75 | 0.75 | 0.75 | 0.75 | 0.75 |
| Hydroxyethyl cellulose (HEC) | ― | ― | 0.75 | 0.28 | 0.28 | 0.28 | 0.28 | 0.75 | 0.75 |
| Mannitol | 2.0 | 2.0 | 2.0 | 2.0 | 2.0 | 2.0 | 0.5 | 2.0 | 2.0 |
| Boric acid | 0.35 | 0.35 | 0.35 | 0.35 | 0.35 | 0.35 | 0.35 | 0.35 | 0.35 |
| Sodium borate | 0.11 | 0.11 | 0.11 | 0.11 | 0.11 | 0.11 | 0.11 | 0.11 | 0.11 |
| Edetate disodium | 0.1 | 0.1 | 0.1 | 0.1 | 0.1 | 0.1 | 0.1 | 0.1 | 0.1 |
| Sodium chloride | 0.09 | 0.09 | 0.09 | 0.09 | 0.45 | 0.09 | 0.09 | 0.09 | 0.09 |
| Polyquad® | 0.001 | 0.001 | 0.001 | 0.001 | 0.001 | 0.001 | 0.001 | ― | ― |
| Sucrose | ― | ― | ― | ― | ― | 2.5 | ― | 2.5 | 2.5 |
| Polyhexamethylene biguanide | ― | ― | ― | ― | ― | ― | ― | 0.0005 | ― |
| BAC | ― | ― | ― | ― | ― | ― | ― | ― | 0.004 |

### Preparation:

Formulations 1 - 9 were prepared as follows. A first solution (Solution A) was prepared by adding 500 mL of warm purified water to a calibrated two liter aspirator bottle equipped with a magnetic stirrer. PVA and hydroxyethyl cellulose were then added to Solution A and the contents dispersed by stirring. After dispersal of the polymers, a filter assembly was attached to the aspirator bottle (142 mm Millipore filter holder with 0.2 µ filter), and this whole apparatus autoclaved at 121°C for 30 minutes. Solution A with the filter assembly attached was then allowed to cool to room temperature with stirring. A second solution (Solution B), was prepared in a 500 mL beaker containing 300 mL of purified water by adding boric acid, sodium borate and mannitol and dissolving the contents by stirring for 25 minutes. Edetate disodium, sodium chloride, preservatives and other osmolality-building agents, as necessary, were added to Solution B and the contents dissolved with stirring. Solution B was then sterile filtered into the aspirator bottle containing Solution A. The pH of the resultant solution was then adjusted and the volume brought to 100% by sterile filtering purified water.

### EXAMPLE 3

The following ophthalmic compositions used according to the present invention may also be prepared using the procedure detailed in Example 2.

| **INGREDIENT** | **FORMULATION (percent by weight)** | | | | | | | | | |
|---|---|---|---|---|---|---|---|---|---|---|
| | **10** | **11** | **12** | **13** | **14** | **15** | **16** | **17** | **18** | **19** |
| PVA | 1.4 | 1.4 | 1.4 | 1.4 | 1.4 | 1.4 | 1.4 | 1.4 | 1.4 | 1.4 |
| Naphazolene HCl | 0.1 | 0.1 | ― | ― | ― | ― | ― | ― | ― | ― |
| Sodium sulfacetamide | ― | ― | ― | 10.0 | ― | ― | ― | ― | ― | ― |
| Fluorometholone | -- | -- | -- | -- | 0.1 | -- | ― | ― | ― | ― |
| Gentamycin sulfate | ― | ― | ― | ― | ― | 0.4 | ― | ― | ― | ― |
| Levobunolol HCl | ― | ― | 0.5 | ― | ― | ― | ― | ― | ― | ― |
| Mydrysone | ― | ― | ― | ― | ― | ― | 1.0 | ― | ― | -- |
| Pilocarpine nitrate | ― | ― | ― | ― | ― | ― | ― | 1.0 | 1.0 | 1.0 |
| Sodium metabisulfite | ― | ― | 0.4 | ― | ― | ― | ― | ― | ― | ― |
| Mannitol | 2.0 | 2.0 | 2.0 | 2.0 | 2.0 | 2.0 | 2.0 | 2.0 | 4.0 | 0.5 |
| Boric acid | 0.35 | 0.35 | 0.35 | 0.35 | 0.35 | 0.35 | 0.35 | 0.35 | 0.35 | 0.5 |
| Sodium borate | 0.11 | 0.11 | 0.11 | 0.11 | 0.11 | 0.11 | 0.11 | 0.11 | ― | ― |
| Sodium chloride | 0.45 | 0.45 | 0.45 | - | 0.45 | 0.45 | 0.45 | 0.45 | ― | ― |
| Edetate disodium | 0.1 | 0.1 | 0.1 | 0.1 | 0.1 | 0.1 | 0.1 | 0.1 | 0.1 | 0.1 |
| BAC | 0.004 | ― | ― | ― | ― | ― | ― | ― | ― | ― |
| Polyquad® | ― | 0.001 | 0.001 | 0.001 | 0.001 | 0.001 | 0.001 | 0.001 | 0.001 | 0.001 |

### EXAMPLE 4

The following is a typical wetting and soaking composition for use in the present invention for use with RGPs.

| **INGREDIENT** | **AMOUNT (wt%)** |
|---|---|
| PVA | 0.75 |
| HEC | 0.38 |
| Boric acid | 0.35 |
| Sodium borate | 0.11 |
| Mannitol | 2.0 |
| Potassium chloride | 0.038 |
| Magnesium chloride | 0.02 |
| Calcium chloride | 0.0154 |
| Sodium chloride | 0.09 |
| Polysorbate 80 | 0.005 |
| Polyquad® | 0.001 |
| NaOH and/or HCl | pH 7.4 |
| Purified water | q.s. |

### Preparation:

In a suitable container containing approximately 30% of the final volume of purified water, PVA and HEC were added and dispersed. This solution was then autoclaved. The solution was allowed to cool to room temperature with stirring. In a separate container, containing approximately 50% of the final volume of purified water, boric acid and sodium borate were added, and dissolved, followed by mannitol. This second solution was then stirred for about 30 minutes, then potassium chloride, calcium chloride, magnesium chloride, sodium chloride, polysorbate 80 and Polyquad® were added, with stirring. The second solution was then added to the first solution via a 0.2 µ filter. Last, the pH was adjusted to 7.4 and the volume brought to 100% with purified water.

### EXAMPLE 5

The following is a typical daily cleaner composition for use in the present invention for use with RGPs and may be prepared in a manner similar to that detailed in Example 4.

| **INGREDIENT** | **AMOUNT (wt%)** |
|---|---|
| Nylon 11 | 2.50 |
| Dextran 70 | 6.0 |
| Sodium borate | 0.25 |
| Boric acid | 0.50 |
| Miracare® 2MCA | 0.50 |
| PDMA-1 | 0.15 |
| Propylene glycol | 10.0 |
| Polyquad® | 0.0055 |
| EDTA | 0.10 |
| Mannitol | 1.20 |
| NaOH and/or HCl | pH 7.4 |
| Purified water | q.s. |

### EXAMPLE 6

The following is a typical wetting and soaking composition for use in the present invention which may be prepared in a manner similar to that detailed in Example 4.

| **INGREDIENT** | **AMOUNT (wt%)** |
|---|---|
| Hydroxypropyl methylcellulose (Methocel® E4M) | 0.72 |
| Mannitol | 1.0 |
| Sodium borate | 0.11 |
| Boric acid | 0.35 |
| Sodium chloride | 0.19 |
| Polyquad® | 0.0011 |
| EDTA | 0.10 |
| NaOH and/or HCl | pH 7.4 |
| Purified water | q.s. |

### EXAMPLE 7

The following is a typical comfort drop composition for use in the present invention which may be prepared in a manner similar to that detailed in Example 4.

| **INGREDIENT** | **AMOUNT (w/v%)** |
|---|---|
| PVA | 0.75 |
| HEC | 0.28 |
| Mannitol | 2.0 |
| Sodium borate | 0.11 |
| Boric acid | 0.35 |
| Sodium chloride | 0.152 |
| Polyquad® | 0.00082 |
| EDTA | 0.10 |
| NaOH and/or HCl | pH 7.4 |
| Purified water | q.s. |

### EXAMPLE 8

The following is a typical RGP cleaner composition for use in the present invention which may be prepared in a manner similar to that detailed in Example 4..

| **INGREDIENT** | **AMOUNT (wt%)** |
|---|---|
| French Naturelle® ES | 2.5 |
| (Nylon 11) | |
| Hydroxyethyl cellulose | 0.4 |
| Sodium borate, decahydrate | 0.25 |
| Boric acid | 0.50 |
| Mannitol | 3.5 |
| Miracare® 2MCA) | 0.50 |
| Isopropyl alcohol (v/v) | 10.0 |
| NaOH and/or HCl | q.s. 7.4 |
| Purified water | q.s. |

### EXAMPLE 9

The following is a typical RGP wetting and/or soaking composition for use in the present invention., which may be prepared in a manner similar to that detailed in Example 4.

| **INGREDIENT** | **AMOUNT (wt%)** |
|---|---|
| Methocel® E4M | 0.85 |
| Mannitol | 2.00 |
| Sodium borate | 0.11 |
| Boric acid | 0.35 |
| Sodium chloride | 0.19 |
| Disodium edetate | 0.1 |
| Polyquad® | 0.001 |
| NaOH and/or HCl | pH 7.4 |
| Purified water | q.s. |

### EXAMPLE 10

The following study compared the antimicrobial preservative efficacy of two wetting, soaking and disinfecting solutions: one containing phosphate buffer (Formulation A); and the other containing a borate-polyol complex of the present invention (Formulation B).

Formulations A and B are shown in the following table.

| **INGREDIENT** | **FORMULATION (wt%)** | |
|---|---|---|
| | **A** | **B** |
| PVA | 0.75 | 0.75 |
| HEC | 0.5 | 0.5 |
| Sodium phosphate | 0.67 | --- |
| Sodium biophosphate | 0.017 | --- |
| Boric acid | --- | 0.35 |
| Sodium borate | --- | 0.11 |
| Mannitol | --- | 2.0 |
| Disodium edetate | 0.1 | 0.1 |
| Sodium chloride | 0.458 | 0.153 |
| Polysorbate 80 | 0.005 | 0.005 |
| Benzalkonium chloride | 0.01 | 0.01 |
| Purified water | q.s. | q.s. |

Formulations A and B were tested against FDA challenge organisms. The log reductions after 1 hour are tabulated below:

| **TEST ORGANISM** | **FORMULATION (log reduction)** | |
|---|---|---|
| | **A** | **B** |
| *A. niger* | 2.1 | 4.4 |
| *B. albicans* | 4.0 | 5.3 |
| *P. aeruginosa* | 5.3 | 5.3 |
| *S. aureus* | 5.5 | 5.2 |
| *E. coli* | 5.5 | 5.5 |

The results shown above indicate that Formulation B (containing borate-polyol complex) has a broader spectrum of activity than Formulation A (containing phosphate buffer), and has greater activity against certain organisms, such as A. *niger.*

### EXAMPLE 11

The following study compared the antimicrobial preservative efficacy of two unpreserved saline solutions identical except that one contained a borate-polyol complex of the present invention (Formulation C) and the other contained the conventional borate buffer (Formulation D).

An organism challenge approach based on the British Pharmacopoeia ("BP") 1988 Test for Efficacy of Preservatives in Pharmaceutical Products was used to evaluate the antimicrobial preservative efficacy of Formulations C and D. Formulation samples were inoculated with known levels of *A. niger* and sampled at predetermined intervals to determine if the system was capable of killing or inhibiting the propagation of organisms introduced into the products.

Formulations C and D are shown in the following table.

| **INGREDIENT** | **FORMULATION (wt%)** | |
|---|---|---|
| | **C** | **D** |
| Boric acid | 1.0 | 1.0 |
| Sodium borate | 0.2 | 0.2 |
| Mannitol | 15 | --- |
| Sodium chloride | --- | 0.3 |
| Disodium edetate | 0.1 | 0.1 |
| NaOH and/or HCl | pH 7.4 | pH 7.4 |
| Purified water | q.s. | q.s. |

The results indicated that there was a 3.1 log reduction of *A. niger* with Formulation C and only 1.2 log reduction with Formulation D after 7 days. Formulation C met the BP standards for preservative efficacy against *A. niger,* while Formulation D failed to meet the BP standards.

### EXAMPLE 12

The following study compared the antimicrobial preservative efficacy of two disinfecting solutions identical except that one contained a borate-polyol complex of the present invention (Formulation E) and the other contained the conventional borate buffer (Formulation F).

An organism challenge approach based on the BP 1988 Test for Efficacy of Preservatives in Pharmaceutical Products was used to evaluate the antimicrobial preservative efficacy of Formulations E and F. Formulation samples were inoculated with known levels of *A. niger* and sampled at predetermined intervals to determine if the system was capable of killing or inhibiting the propagation of organisms introduced into the products.

Formulations E and F are shown in the following table.

| **INGREDIENT** | **FORMULATION (wt%)** | |
|---|---|---|
| | **E** | **F** |
| Boric acid | 0.3 | 0.35 |
| Sodium borate | 0.11 | 0.11 |
| Mannitol | 0.85 | --- |
| Sodium citrate | 0.56 | 0.56 |
| Citric acid | 0.021 | 0.21 |
| Sodium chloride | 0.48 | 0.48 |
| Pluronic P103 | 0.5 | 0.5 |
| Disodium edetate | 0.05 | 0.05 |
| Polyquad® | 0.001 | 0.001 |
| NaOH and/or HCl | pH 7.0 | pH 7.0 |
| Purified water | q.s. | q.s. |

The results indicate that there was a 2.1 log reduction of *A. niger* with Formulation E and only 1.1 log reduction with Formulation F after 7 days. Formulation E met the BP standards for preservative efficacy against *A. niger,* while Formulation F failed to meet the BP standards.

The invention has been described by reference to certain preferred embodiments; however, it should be understood that it may be embodied in other specific forms or variations thereof without departing from its spirit or essential characteristics. The embodiments described above are therefore considered to be illustrative in all respects and not restrictive, the scope of the invention being indicated by the appended claims rather than by the foregoing description.

## Claims

1. Use of a water-soluble borate-polyol complex, said polyol having at least two adjacent -OH groups which are not in trans configuration relative to each other, as an ophthalmic antimicrobial agent in an aqueous ophthalmic composition, wherein the polyol is selected from the group consisting of mannitol, glycerin, propylene glycol and sorbitol.

2. Use of a water-soluble borate-polyol complex, said polyol having at least two adjacent -OH groups which are not in trans configuration relative to each other, as an ophthalmic antimicrobial agent against *A. niger* in an aqueous composition.

3. Use according to claims 1 or 2, wherein the borate-polyol complex is included in an aqueous ophthalmic composition for treating contact lenses.

4. Use according to any of claims 1 to 3, wherein borate is selected from the group consisting of boric acid, sodium borate, potassium borate, calcium borate and magnesium borate.

5. Use according to claim 2, wherein the polyol is selected from the group consisting of mannitol, glycerin, propylene glycol and sorbitol.

6. Use according to any of claims 1 or 5, wherein the polyol is mannitol.

7. Use according to any of claims 1 or 5, wherein the polyol is glycerin.

8. Use according to any of claims 1 or 5, wherein the polyol is sorbitol.

9. Use according to claim 1 or 2, wherein the borate-polyol complex is employed together with a viscosity-enhancing polymer.

10. Use according to claim 9, wherein the polymer is polyvinyl alcohol, a cellulosic polymer, or a carboxy vinyl polymer.

11. Use according to claim 9 or 10, wherein the polymer is a cellulosic polymer.

12. Use according to any of claims 1 to 11, wherein 0.5 to 6.0 wt% of the water-soluble borate-polyol complex is included in an aqueous ophthalmic composition, the complex containing borate and polyol in a molar ratio of 1:0.1 to 1:10.

13. Use according to claim 12, wherein the borate-polyol complex is included in the composition at a concentration of 1.0 to 2.5 wt%, and the molar ratio of borate to polyol is 1:0.25 to 1:2.5.

14. Use according to any of claims 1 to 13, wherein the borate-polyol complex is employed together with an ophthalmically acceptable antimicrobial agent.

15. Use according to claim 14, wherein the antimicrobial agent is selected from monomeric and polymeric quaternary ammonium compounds and their ophthalmically acceptable salts, monomeric and polymeric biguanides and their ophthalmically acceptable salts, and combinations thereof.

16. Use according to claim 15, wherein the antimicrobial agent is polyquaternium-1 or polyhexamethylene biguanide.

17. Use according to claim 15, wherein the antimicrobial agent is polyqaternium-1.

18. Use according to claim 15, wherein the antimicrobial agent is polyhexamethylene biguanide.

19. Use according to any of claims 1 to 18, wherein the borate-polyol complex is included in an aqueous ophthalmic composition which is a solution for disinfecting contact lenses, to enhance the antimicrobial activity of the solution.

20. Use according to any of claims 1 to 13, wherein the borate-polyol complex is included in an aqueous ophthalmic composition which is an unpreserved saline solution for treating contact lenses, to enhance the antimicrobial activity of the saline solution.

## Patentansprüche

1. Verwendung eines wasserlöslichen Borat-Polyol-Komplexes, wobei das Polyol mindestens zwei nebeneinanderliegende -OH-Gruppen aufweist, die nicht in trans-Konfiguration zu einander stehen, als ophthalmisches antimikrobielles Mittel in einer wässrigen ophthalmischen Zusammensetzung, wobei das Polyol aus der Gruppe ausgewählt wird, die aus Mannit, Glycerin, Propylenglykol und Sorbit besteht.

2. Verwendung eines wasserlöslichen Borat-Polyol-Komplexes, wobei das Polyol mindestens zwei nebeneinanderliegende -OH-Gruppen aufweist, die nicht in trans-Konfiguration zu einander stehen, als ophthalmisches antimikrobielles Mittel gegen *A. niger* in einer wässrigen Zusammensetzung.

3. Verwendung nach Anspruch 1 oder 2, wobei der Borat-Polyol-Komplex in einer wässrigen ophthalmischen Zusammensetzung für die Behandlung von Kontaktlinsen enthalten ist.

4. Verwendung nach einem der Ansprüche 1 bis 3, wobei das Borat aus der Gruppe ausgewählt wird, die aus Borsäure, Natriumborat, Kaliumborat, Calciumborat und Magnesiumborat besteht.

5. Verwendung nach Anspruch 2, wobei das Polyol aus der Gruppe ausgewählt wird, die aus Mannit, Glycerin, Propylenglykol und Sorbit besteht.

6. Verwendung nach einem der Ansprüche 1 oder 5, wobei das Polyol Mannit ist.

7. Verwendung nach einem der Ansprüche 1 oder 5, wobei das Polyol Glycerin ist.

8. Verwendung nach einem der Ansprüche 1 oder 5, wobei das Polyol Sorbit ist.

9. Verwendung nach einem der Ansprüche 1 bis 2, wobei der Borat-Polyol-Komplex zusammen mit einem die Viskosität verbessernden Polymer verwendet wird.

10. Verwendung nach Anspruch 9, wobei das Polymer Polyvinylalkohol, ein Cellulosepolymer oder ein Carboxyvinyl-Polymer ist.

11. Verwendung nach Anspruch 9 oder 10, wobei das Polymer ein Cellulosepolymer ist.

12. Verwendung nach einem der Ansprüche 1 bis 11, wobei 0,5 bis 6,0 Gew.-% des wasserlöslichen Borat-Polyol-Komplexes in einer wässrigen ophthalmischen Zusammensetzung enthalten ist, wobei der Komplex Borat und Polyol in einem Molverhältnis von 1:0,1 bis 1:10 enthält.

13. Verwendung nach Anspruch 12, wobei der Borat-Polyol-Komplex in der Zusammensetzung in einer Konzentration von 1,0 bis 2,5 Gew.-% enthalten ist und das Molverhältnis von Borat zu Polyol 1:0,25 bis 1:2,5 beträgt.

14. Verwendung nach einem der Ansprüche 1 bis 13, wobei der Borat-Polyol-Komplex zusammen mit einem ophthalmisch akzeptablen antimikrobiellen Mittel verwendet wird.

15. Verwendung nach Anspruch 14, wobei das antimikrobielle Mittel unter monomeren und polymeren quartären Ammoniumverbindungen und ihren ophthalmisch akzeptablen Salzen, monomeren und polymeren Biguaniden und ihren ophthalmisch akzeptablen Salzen und Kombinationen derselben ausgewählt wird.

16. Verwendung nach Anspruch 15, wobei das antimikrobielle Mittel Polyquaternium-1 oder Polyhexamethylenbiguanid ist.

17. Verwendung nach Anspruch 15, wobei das antimikrobielle Mittel Polyquaternium-1 ist.

18. Verwendung nach Anspruch 15, wobei das antimikrobielle Mittel Polyhexamethylenbiguanid ist.

19. Verwendung nach einem der Ansprüche 1 bis 18, wobei der Borat-Polyol-Komplex in einer wässrigen ophthalmischen Zusammensetzung enthalten ist, die eine Lösung für das Desinfizieren von Kontaktlinsen ist, um die antimikrobielle Aktivität der Lösung zu erhöhen.

20. Verwendung nach einem der Ansprüche 1 bis 13, wobei der Borat-Polyol-Komplex in eine wässrigen ophthalmischen Zusammensetzung , bei der es sich um eine nichtkonservierte physiologische Kochsalzlösung für die Behandlung von Kontaktlinsen handelt, zum Erhöhen der antimikrobiellen Aktivität der physiologischen Kochsalzlösung eingearbeitet ist.

## Revendications

1. Utilisation d'un complexe de borate-polyol soluble dans l'eau, ledit polyol ayant au moins deux groups -OH adjacents qui ne sont pas en transconfiguration l'un par rapport à l'autre, en tant qu'agent antimicrobien ophtalmique dans une composition ophtalmique aqueuse, où le polyol est sélectionné parmi le groupe composé de mannitol, de glycérine, de glycol propylène et de sorbitol.

2. Utilisation d'un complexe de borate-polyol soluble dans l'eau, ledit polyol ayant au moins deux groups -OH adjacents qui ne sont pas en transconfiguration l'un par rapport à l'autre, en tant qu'agent antimicrobien ophtalmique contre le microbe *A. niger* dans une composition ophtalmique aqueuse.

3. Utilisation selon la revendication 1 ou 2, où le complexe de borate-polyol est inclus dans une composition ophtalmique aqueuse pour le traitement des lentilles de contact.

4. Utilisation selon l'une quelconque des revendications 1 à 3, où le borate est sélectionné par le groupe composé d'acide borique, de borate de sodium, de borate de potassium, de borate de calcium et de borate de magnésium.

5. Utilisation selon la revendication 2, où le polyol est sélectionné parmi le groupe composé de mannitol, de glycérine, de glycol propylène et de sorbitol.

6. Utilisation selon l'une quelconque des revendications 1 ou 5, où le polyol est le mannitol.

7. Utilisation selon l'une quelconque des revendications 1 ou 5, où le polyol est la glycérine.

8. Utilisation selon l'une quelconque des revendications 1 ou 5, où le polyol est le sorbitol.

9. Utilisation selon l'une quelconque des revendications 1 ou 2, où le complexe de borate-polyol est employé avec un polymère renforçant la viscosité.

10. Utilisation selon la revendication 9, où le polymère est de l'alcool de polyvinyle, un polymère de cellulose ou un polymère de carboxy vinyle.

11. Utilisation selon la revendication 9 ou 10, où le polymère est un polymère de cellulose.

12. Utilisation selon l'une quelconque des revendications 1 à 11, où 0,5 à 6,0 % en poids du complexe de borate-polyol soluble dans l'eau est inclus dans une composition ophtalmique aqueuse, le complexe contenant du borate et du polyol dans un rapport molaire de 1:0,1 à 1:10.

13. Utilisation selon la revendication 12, où le complexe de borate-polyol est inclus dans la composition à une concentration de 1,0 à 2,5 % en poids et où le rapport molaire du borate au polyol est compris entre 1:0,25 et 1:2,5.

14. Utilisation selon l'une quelconque des revendications 1 à 13, où le complexe de borate-polyol est employé avec un agent antimicrobien acceptable du point de vue ophtalmique.

15. Utilisation selon la revendication 14, où l'agent antimicrobien est sélectionné parmi des composés d'ammonium quartenaire monomères et polymères et leurs sels acceptables du point de vue ophtalmique, les biguanides monomères et polymères ainsi que les sels acceptables du point de vue ophtalmique, et leurs combinaisons.

16. Utilisation selon la revendication 15, où l'agent antimicrobien est du polyquaternaire-1 ou du biguanide polyhexaméthylène.

17. Utilisation selon la revendication 15, où l'agent antimicrobien est du polyquaternaire-1.

18. Utilisation selon la revendication 15, où l'agent antimicrobien est du biguanide de polyhexaméthylène.

19. Utilisation selon l'une quelconque des revendications 1 à 18, où le complexe de borate-polyol est inclus dans une composition ophtalmique aqueuse qui constitue une solution saline sans agent conservateur pour le traitement des lentilles de contact, dans le but d'améliorer l'activité antimicrobienne de la solution.

20. Utilisation selon l'une quelconque des revendications 1 à 13, où le complexe de borate-polyol est inclus dans une composition ophtalmique aqueuse constituant une solution sans agent conservateur pour le traitement des lentilles de contact, dans le but d'améliorer l'activité antimicrobienne de la solution saline.
